(19) Europäisches Patentamt / European Patent Office / Office européen des brevets

(11) **EP 4 116 701 A1**

(12) **EUROPEAN PATENT APPLICATION**

(43) Date of publication:
**11.01.2023 Bulletin 2023/02**

(21) Application number: **21183799.2**

(22) Date of filing: **05.07.2021**

(51) International Patent Classification (IPC):
**G01N 21/35** (2014.01)          **G01N 21/03** (2006.01)
**G01N 33/00** (2006.01)          **G01N 33/22** (2006.01)
**G01J 3/26** (2006.01)

(52) Cooperative Patent Classification (CPC):
**G01N 21/031; G01J 3/26; G01N 33/0036;**
G01N 21/3504; G01N 33/0047; G01N 33/005;
G01N 33/225; G01N 2201/0221

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität Wien**
**1010 Wien (AT)**

(72) Inventor: **The designation of the inventor has not yet been filed**

(74) Representative: **J A Kemp LLP**
**80 Turnmill Street**
**London EC1M 5QU (GB)**

(54) **GAS-SENSING APPARATUS**

(57)    A gas-sensing apparatus is provided. The gas-sensing apparatus comprises a test chamber formed in a body and comprising a pair of micromirrors. One of the pair of micromirrors is disposed on a first surface of the body and the other of the pair of micromirrors is disposed on a second surface of the body, forming an optical cavity. A light inlet is arranged to couple light into the optical cavity, and light outlets are arranged to receive light from the optical cavity. A gas inlet configured to allow gas from outside of the detector to enter the test chamber. A gas detector comprising a gas-sensing apparatus, a light emitting system, and a light detecting system is also provided.

Fig. 1

**Description**

The present invention relates to a gas-sensing apparatus, a gas detector comprising a gas-sensing apparatus, and a method of detecting a target gas species in an environment.

There are many situations where it is desirable to detect and monitor one or more target gasses in an environment. One example is monitoring of atmospheric methane pollution and gas leaks. Methane ($CH_4$ or natural gas) has an 80 times higher 20-year global warming potential (GWP-20) compared to $CO_2$ and more than 60% of the global $CH_4$ emissions are manmade. One of the biggest sources are abandoned oil and gas wells. The number of leaking wells is estimated to be 30-40 million worldwide, some of which are more than 100 years old. The total leakage is on the percent level of the annual natural gas production. This is a global threat which must be tackled to stop global warming. However, burning methane produces only half as much $CO_2$ as burning coal. Hence natural gas is considered as a bridge energy source, but emissions must be avoided to benefit from replacing methane with coal. Monitoring of methane leaks will be a key part of meeting climate goals. Current solutions, however, are costly and have a low sensitivity.

An additional area where gas sensing is important is detecting excess hydrocarbons in fluids such as hydrogen. Fuel cells are believed to be a part of the energy revolution needed to combat climate change. They use hydrogen and could power trucks, planes, and ships in the future. The efficiency and lifetime of fuel cells depends on the durability of the electrodes. Any contamination in the fuel will degrade efficiency and lifetime and therefore is a major problem. Hydrogen stations normally use extremely expensive sensors, which usually rely on mass spectrometers, to monitor fuel quality.

Additionally, detection of refrigerant leaks in the refrigeration cycle of air conditioning/cooling systems and heat pumps will become a growing necessity since fugitive refrigerants have an extremely high global warming potential.

Trace gas detection is also an emerging technique in human health monitoring by breath gas analysis. Detection of volatile organic compounds is used for diagnosing early stages of serious medical conditions, like breast cancer or small intestinal bacterial overgrowth. Small and affordable personal health monitors will be a fast-growing market and field of applications in the future.

Gas detection can be performed in a range of devices which rely on different physical effects. Optical methods are superior but are difficult to technically implement in high precision applications. Optical absorption allows the direct measurement and identification of the concentration of chemical molecules in air or any other host atmosphere, without using any proxy or catalytic effect. Many chemical substances (e.g. hydrocarbons) absorb light in the near- to mid-infrared (NIR to MIR) wavelength domain. Fundamental absorption bands, which naturally give the strongest absorption signal, are found in the MIR range. However, operating at MIR wavelengths comes at an extremely high cost for light sources and detectors. Hence such devices are powerful but not suitable for field use and large-scale deployment, due to cost and technical overhead.

Detection of gas molecules at higher absorption overtones in the NIR is used to avoid working in the expensive and unpractical MIR range. The biggest advantage is that NIR coincides with the optical telecom wavelength domain, where low-cost components and highly sophisticated manufacturing techniques, lasers and detectors are available. Most NIR sensors are compact, non-diffractive gas sensors which measure infrared absorption in single pass cells, which already fit on printed circuit boards. However commercially available sensors are only able to detect methane concentrations down to some few hundreds of parts per million (ppm).

The natural concentration of methane in air is 1.87 ppm i.e. two orders of magnitude lower than the lower detection limit of compact state of the art NIR sensors. This is useful for only a limited number of applications, e.g. explosion hazard warning systems, but not for monitoring any critical environment where small concentrations need to be detected, such as methane detection from gas leaks.

Current optical remote gas sensors are mostly single pass devices which have effective absorption lengths $l$ of only a few tens of centimetre. The strength of the detectable absorption signal is directly proportional to the attenuation coefficient $\alpha$ and the absorption length $l$. The absorption length is the distance the light travels through the medium before it hits a detector. The attenuation coefficient is the quantity to be measured and is related to the concentration of trace gas molecules in the atmosphere. The relation between incident $I$ and the transmitted $I_0$ light intensities is expressed by the Beer-Lambert law as:

$$I = I_0 e^{\alpha l}$$

with the absorption length $l$ and the absorption coefficient $\alpha = \varepsilon c$. Here $\varepsilon$ is the molar attenuation coefficient and $c$ the concentration of the absorbing molecule in the atmosphere. For naturally abundant (1.87 ppm) methane the absorption coefficient at 1650 nm is $\alpha \approx$ 1x10-6 cm-1. For an absorption length of 10 cm and a laser intensity of 10 mW the change in laser intensity would only be on the order of 50 nW which means a relative change of only 5 ppm in laser intensity and requires more than 50 dB of dynamical range. It is unrealistic to detect such small relative signals in any practical system where noise is present. The major sources of noise are: laser intensity fluctuations and frequency instability; electronic readout noise and mechanical vibrations of the optical system. Thus, for gas-sensing applications such as methane detection and hydrogen fuel monitoring, current solutions do not provide the combination of high sensitivity and low cost required.

It is an object of the invention to improve gas sensing and/or at least partially address one or more of the challenges with the prior art described above.

According to a first aspect of the invention there is provided a gas-sensing apparatus comprising: a test chamber formed in a body, the test chamber comprising a pair of micromirrors, one of the pair of micromirrors being disposed on the first surface of the body and the other of the pair of micromirrors being disposed on the second surface of the body, wherein the pair of micromirrors forms an optical cavity; a light inlet arranged to couple light into the optical cavity; light outlets arranged to receive light from the optical cavity; and a gas inlet configured to allow gas from outside of the detector to enter the test chamber.

A micromirror may be a flat or curved surface arranged to reflect optical beams with waists of 500 $\mu$m or less, or 100 $\mu$m or less. A maximum size of the micromirror (e.g. width or diameter) may be 500 $\mu$m or less, or 100 $\mu$m or less.

The micromirrors allow the apparatus to be formed by micro-fabrication. Such a micro-fabricated device can provide high sensitivity detection in a small, light-weight device. The optical cavity increases the effective absorption length *l* of the apparatus, increasing the sensitivity of the apparatus. At the same time, thermal noise is reduced compared to conventional systems, as the physically small cavity experiences only minimal thermal expansion. Thus the use of an optical cavity and use of micron-scale mirrors combine to achieve higher sensitivities, enabling the apparatus to be used in applications such as methane detection where high sensitivity is essential. Indeed, the present inventors have found that the sensitivity limit of such a gas-sensing apparatus may be three orders of magnitude higher than conventional compact optical gas sensors.

Thus the gas-sensing apparatus may be used as part of a portable (e.g. handheld) gas detector. The gas-sensing apparatus may be particularly suited to detection of hydrocarbons, such as methane. In particular, it may outperform conventional technologies in terms of cost and sensitivity for detection of methane leaks. It may also be useful for detecting excess hydrocarbons in hydrogen. Due its relatively low cost and small size, the gas-sensing apparatus may be incorporated into a hydrogen fuel cell, providing on-board monitoring of fuel quality.

In some embodiments the gas-sensing apparatus may be part of an air conditioning/cooling system or heat pump. The apparatus may be used to detect refrigerant leaks from a refrigeration cycle of the unit. Such an implementation may be required by state regulators in the future. Such applications benefit from the low cost, small form-factor apparatus possible in the present invention.

In some embodiments the gas-sensing apparatus may be, or may be part of, a personal health monitoring system configured to detect one or more gases in human breath. For example, breath gas analysis by measuring of methane and volatile organic compounds (VOC) in human breath may be used for the detection of early stages of diseases. Alternatively the analysis may be used for the monitoring and control of breath gases during anesthesia.

In some embodiments, the optical cavity is a Fabry-Perot optical cavity. A Fabry-Perot cavity is particularly suited to gas sensing, providing large effective absorption lengths relative to the physical length of the cavity, and so increasing the sensitivity of the apparatus.

In some embodiments, the body comprises a first part and a second part, the test chamber being formed between the first part and the second part, and wherein the first surface is a surface of the first part, and the second surface is a surface of the second part. The first part may be a first substrate and the second part may be a second substrate, and wherein the body may further comprise a spacing structure separating the first substrate and the second substrate. Advantageously, the first part, second part, and/or spacing structure may be formed from silicon wafers. Very high precision manufacturing processes are available for silicon, allowing high quality, small scale optical cavities to be formed. Alternatively any other semiconductor wafer, or more generally any suitable material, may be used.

In some embodiments, the test chamber comprises a plurality of pairs of micromirrors, one of each pair of micromirrors being disposed on the first surface and the other of each pair of micromirrors being disposed on the second surface, wherein each pair of micromirrors forms a respective optical cavity. The light inlet is arranged to couple light into one or more of the optical cavities. The light outlet is arranged to receive light from one or more of the optical cavities.

As the optical cavities are formed from micromirrors, a large number of optical cavities may be provided, all within a small form-factor apparatus. Advantageously, the optical cavities may have different resonant frequencies, allowing the gas-sensor to detect multiple target gas species, and/or to detect multiple absorption peaks from the same target gas.

In some embodiments, the apparatus further comprises a reference chamber formed in the body, the reference chamber comprising: one or more pairs of micromirrors, each pair of micromirrors forming a reference optical cavity; a light inlet arranged to couple light into one or more of the reference optical cavities; and a light outlet arranged to receive light from one or more of the reference optical cavities; wherein the reference cavity is sealed or is sealable from outside gasses.

The reference chamber allows balanced detection techniques to be used to further enhance the sensitivity of the device. Due to the small size of the optical cavities, the reference chamber is physically close to the test chamber. Therefore any excess noise, such as mechanical, laser, and electronic noise, is likely to be very similar for the reference and test chambers, allowing noise to be cancelled more effectively.

In some embodiments the apparatus further comprises an optical cavity tuning system configured to alter the resonant frequency of one or more of the optical cavities. In some such embodiments the optical cavity tuning system is configured to change the temperature of the micromirrors and/or the body (or parts thereof, e.g. the spacing structure) in order to vary the resonant frequency of one or more of the optical cavities. Alternatively or additionally, the optical cavity tuning system may be configured to apply a piezoelectric-controlling signal to a piezoelectric material of the micromirrors and/or the body (or parts thereof e.g. the spacing structure) in order to vary the resonant frequency of the one or more of the optical cavities. In particular embodiments, all optical cavities are tuned simultaneously. Where the apparatus comprises a reference chamber, the reference optical cavities may also be tuned.

The optical cavity tuning system allows the resonant frequencies of the optical cavities to be varied across a particular range. This allows absorption measurements that scan across a range of frequencies, for example scanning around an absorption peak of a target gas. This may provide greater certainty in identification of a gas compared to detecting a single frequency.

In some embodiments, the optical cavity tuning system is arranged to monitor the resonant frequencies of one or more the optical cavities, and to alter the resonant frequency of one or more of the optical cavities based on the monitored resonant frequencies. Such embodiments enable the optical cavities to be dynamically controlled to account for variations, e.g. due to thermal fluctuations, further enhancing the sensitivity of the apparatus.

In some embodiments, the optical cavity or optical cavities are configured to have resonances in the visible to near-infrared electromagnetic ranges, or in the near-infrared electromagnetic range. This range match the ranges used by telecoms devices. Laser sources and detectors used in telecoms devices tend to be small, extremely power efficient, and low cost. Thus by using these frequency ranges the apparatus can be constructed from easily sourced components that allow the apparatus to be smaller, cheaper, and more portable.

According to a second aspect of the invention there is provided a gas detector comprising: the gas-sensing apparatus of any embodiment of the first aspect; a light emitting system arranged to transmit light into the light inlet of the gas-sensing apparatus; and a light detecting system arranged to receive light from the light outlets of the gas-sensing apparatus, wherein the light detecting system is configured to generate a signal representative of the intensity of the received light.

In some embodiments, the light emitting system comprises a light source and an optical fibre, the optical fibre arranged to couple light from the light source into the light inlet. In some embodiments the light detecting system comprises one or more photodiodes and one or more optical fibres, the one or more optical fibres arranged to couple light from the light outlet onto the one or more photodiodes. Such embodiments may be useful for remote sensing applications. A common light source and/or detector may be coupled by fibres to multiple detectors. For example, multiple detectors may be positioned around an oil well to detect methane leaks. This can provide a cost effective monitoring solution, as only one light source (e.g. laser) and/or detector is required.

In some embodiments the light emitting system and/or light detecting system is incorporated into the gas-sensing apparatus. For example, an LED or laser diode may be incorporated into the first part, and a photodiode detector may be incorporated into the second part. Such embodiments may provide a complete gas detector in a small and light-weight form.

According to a third aspect of the invention there is provided a method of detecting presence of a target gas species in an environment, the method comprising: positioning a gas-sensing apparatus according to any embodiment of the first aspect in the environment such that gasses from the environment enter the test chamber of the apparatus; inputting a light beam into an optical cavity of the gas-sensing apparatus; detecting the light exiting the optical cavity; and analysing the detected light to determine whether the target gas species is present.

According to a fourth aspect of the invention there is provided a method of providing a gas-sensing apparatus for use in detecting presence of a target gas species, the method comprising: constructing the gas-sensing apparatus by forming a test chamber between a first part and a second part, the test chamber comprising a plurality of pairs of micromirrors, one of each pair of micromirrors being disposed on the first part and the other of each pair of micromirrors being disposed on the second part, wherein each pair of micromirrors forms a respective optical cavity; coupling light into each optical cavity to determine a resonant frequency of each optical cavity; comparing the determined resonance frequencies to the frequency of an absorption peak of the target gas species; selecting one of the plurality of optical cavities based on the comparison of resonance frequencies to the frequency of the absorption peak; and configuring the gas-sensing apparatus to detect light from the selected optical cavity.

Embodiments will now be described, by way of example only, with reference to the accompanying drawings in which:

EP 4 116 701 A1

Fig. 1 illustrates an example gas-sensing apparatus;

Fig. 2 shows an exploded view of a gas-sensing apparatus;

Fig. 3 illustrates a gas-sensing device comprising a gas-sensing apparatus;

Fig. 4 shows the mode spectra for a Fabry-Perot optical cavity;

Fig. 5 shows the absorption coefficient of methane at 1650nm, and illustrates changes to the resonance condition caused by thermal expansion of a Si chamber;

Figs. 6a and 6b shows absorption coefficients for methane, carbon dioxide, and water;

Fig. 7 illustrates a method of providing a gas-sensing apparatus;

Fig. 8 illustrates a wafer used to manufacture a plurality of gas-sensing apparatus;

Fig. 9 illustrates each layer of a gas-sensing apparatus formed from a die of the wafer of Fig. 8;

Fig. 10 illustrates a method of using a gas-sensing apparatus to determine presence of a target gas;

Fig. 11(a) illustrates use of multiple independent gas-sensing devices arranged around a well pad;

Fig. 11(b) illustrates use of a gas-sensing device comprising a plurality of gas-sensing apparatus arranged around a well pad.

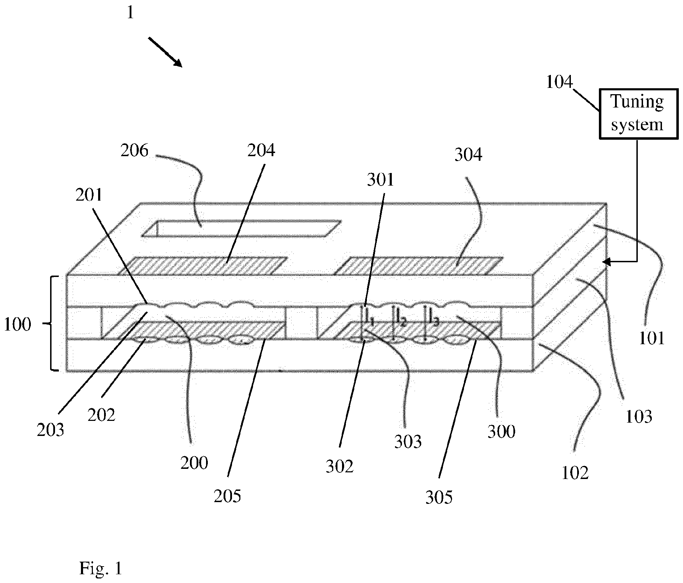

Fig. 1 illustrates an example of a gas-sensing apparatus 1. Gas-sensing apparatus 1 may be configured for sensing/detecting one or more target gas species. The apparatus 1 may be configured to detect methane, for example for use in monitoring methane leaks. The apparatus 1 may be configured to detect hydrocarbons (or one or more specific hydrocarbons). Such cases may be used to monitor purity of hydrogen fuel. Due to the small size and low cost of the apparatus 1, the apparatus 1 may be built directly into a fuel cell. This would provide on-board monitoring of fuel quality, rather than the limited sampling available currently at fuel pumps. The apparatus 1 may be used in a portable (e.g. handheld) gas monitor for detecting one or more desired gases. The apparatus 1 may be used to detect carbon dioxide and or/ carbon monoxide. In general the gas sensing apparatus 1 may be used to detect any desired gas or combination of gases having an absorption feature in a suitable wavelength range, for example in the visible or near-infra red ranges.

Gas-sensing apparatus 1 comprises a test chamber 200 formed in a body 100. The test chamber 200 takes the form of a cavity or open space partially enclosed by the body 100. The test chamber 200 is able to receive gases from outside the apparatus 1, in order to test for the presence of one or more target gases.

The body 100 may comprise a first part 101 and a second part 102. The test chamber 200 is then formed between the first part 101 and the second part 102. In the illustrated example, the first part 101 is a first substrate and the second part 102 is a second substrate. The body 100 further comprises a spacing structure 103 separating the first substrate and the second substrate. Advantageously, the first and second parts 101, 102 may be formed from standard semiconductor substrate materials, such as silicon. Features of the apparatus 1 can then be formed on the substrates using the highly precise manufacturing processes available for semiconductor manufacturing. Silicon is also a rigid material with a low thermal expansion coefficient, which improves the quality of detection provided by the apparatus 1. Each of the first part 101 and second part 102 may have a thickness of 250 $\mu$m or more, and/or of 1000 $\mu$m or less.

The spacing structure 103 between the first and second parts 101, 102 provides a physical distancing between the opposing surfaces of the first and second parts 101, 102. This spacing sets the height of the test chamber 200. The spacing structure 103 thus defines, on a coarse scale, the length of optical cavities in the test chamber 200, discussed further below. The spacing structure 103 may be made out of silicon, quartz, silicon carbide, or any other suitable material. In common with the first part 101 and second part 102, the spacing structure 103 may be formed from a semiconductor wafer. As discussed further below, the spacing structure, or parts thereof, may be piezoelectric, enabling tuning of the spacing by an applied voltage.

The first part 101, second part 102, and spacing structure 103 may be joined by robust wafer bonding processes, which ensures precise alignment and permanent stability.

The apparatus 1 further comprises a gas inlet 206 configured to allow gas from outside of the apparatus 1 to enter the test chamber 200. In the illustrated example, the gas inlet 206 is formed by an opening in the first part 101. The opening allows outside gas to enter the test chamber 200, so that the gas can be tested for presence of one or more target gases. Alternatively or additionally, gas inlets 206 may pass through the second part 102 and/or spacing structure 103.

Some examples of the apparatus 1 further comprise a reference chamber 300 formed in the body 100. Unlike the open test chamber 200, the reference chamber 300 is closed (or sealable) to outside gases. Instead, the reference chamber 300 is filled, or is fillable with, one or more reference gas species. The reference chamber 300 allows reference measurements to be taken. A comparison of reference measurements taken from reference chamber 300 with test measurements taken from test chamber 200 allows noise to be reduced in the test measurements, and so the accuracy of the gas sensing of the apparatus 1 to be improved. In particular, using the reference chamber 300 may allow balanced detection methods to be employed.

The reference gas/es may include the target gas/es selected as targets for detection in the test chamber 200. Alternatively or additionally, the reference gas/es may include gases other than those selected as targets for detection in the test chamber 200. The reference gases may be injected into the reference chamber 200 during fabrication of the device 1. Alternatively the apparatus 1 may comprise a sealable injection port to allow reference gases to be sealable injected into the reference chamber 300 after fabrication. The sealable injection port may be similar to the gas inlet 206, but is closed after the apparatus 1 has been placed in an atmosphere of the intended reference gases. The reference gases may include one or more gases of defined concentration in a host matrix, such as nitrogen. The reference gasses may include multiple species with well isolated absorption features (e.g. peaks in absorption coefficient). This would allow one reference chamber 300 to be used in multi gas-sensing. Alternatively multiple reference chambers 300 may be used, each corresponding to a respective target gas.

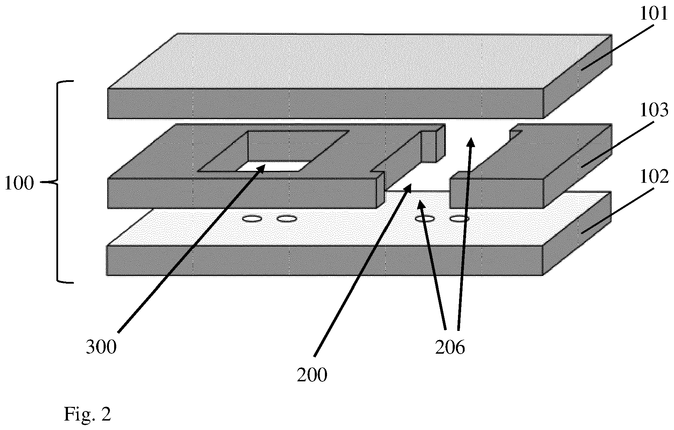

In the illustrated example, the reference chamber 300 is formed between the first part 101 and the second part 102. The reference chamber is substantially similar to the test chamber 200, but without a gas inlet 206. Fig. 2 shows an alternative example of apparatus 1, illustrating the closed nature of the reference chamber 300. In this example, gas inlets 206 to the test chamber 200 are formed through the spacing structure 103.

The gas sensing apparatus 1 is configured to allow absorption of light in the test chamber 200 to be measured. To this end, the test chamber 200 comprises one or more pairs of micromirrors 201, 202. A first micromirror 201 of each pair is disposed on a first surface of the body 100. In the illustrated examples, the first surface is a surface of the first part 101 (i.e. the surface of the first part 101 facing into the test chamber 200). The other micromirror 202 of the pair is disposed on the second surface of the body 1. The second surface is a surface of the second part 102 (i.e. the surface of the second part 102 facing into the test chamber 200). Together, each pair of micromirrors 201, 202 forms an optical cavity 203. The optical cavities 203 may have a (physical) length of 1000 $\mu$m or less, or preferably 500 $\mu$m or less. The optical cavities 203 may thus be considered to be micro-cavities.

The micromirrors 201, 202 are flat or curved surfaces which are wide or big enough to reflect light beams with beam waists smaller than a predefined size, such as 500 $\mu$m or 100 $\mu$m. The micromirrors 201, 202 can thus be small compared to conventional optical cavities, reducing the overall size of the apparatus 1.

The micromirrors 201, 202 may be formed by reactive ion etching techniques, or other high precision techniques. This allows the length of the optical cavities 203 to be very precisely controlled. Control on the scale of tens of nanometer or better is achievable. Precise knowledge of the length of the optical cavity 203 is important for setting the resonant frequency of the optical cavity 203, and for correctly determining the amount of absorption of light in the optical cavity 203.

In some examples, the test chamber 200 comprises a plurality of pairs of micromirrors 201, 202. In particular, the test chamber 200 may comprise one or more pairs of micromirrors 201, 202 for each of one or more target gas species to be detected by the gas-sensing apparatus. Each pair of micromirrors 201, 202 may be configured to have a different resonance frequency. The different resonance frequencies may be selected to match target absorption features (e.g. peaks in the absorption coefficient) of the different target species. Alternatively or additionally, the different resonance frequencies may be selected to correspond to different absorption features of the same target gas. In some examples, the test chamber 200 (and optionally reference chamber 300) may be fabricated with a plurality of micromirrors 201, 202 of differing resonance frequencies. One or more pairs of micromirrors 201, 202 may then be selected post-fabrication, based on the intended target gas or gases. In other words, the apparatus 1 may be initially fabricated to be target gas-neutral.

In some examples, the test chamber 200 may comprise 2 or more, or 5 or more, or 10 or more pairs of micromirrors for each of the one or more target gas species. In some examples, the gas-sensing apparatus 1 may be configured to detect 2 or more, or 5 or more, or 10 or more target gas species. Thus in some examples, the target chamber 200 may comprise 100 or more optical cavities 203.

In some examples, the micromirrors 201, 202 are coated with high-reflectivity multilayer coatings. The mirror shape is first created by structuring of the respective substrate 101, 102. The shaped substrate 101, 102 is then coated with high-reflectivity coatings. For example, multilayer dielectric Bragg coatings may be applied by sputtering or another suitable process. The center wavelength of the coatings can be freely chosen from the visible to near infrared electromagnetic regions. Coatings typically work on bandwidths of 50-100 nm. Where the test chamber 200 comprises multiple optical cavities 203, the center wavelength each respective pair of micromirrors 201, 202 may differ.

To detect a target gas, light is coupled into the one or more optical cavities 203, where it is absorbed by the gas in the test chamber 200. To this end, the apparatus 1 comprises a light inlet 204 arranged to couple light into the one or more optical cavities 203. Light that is resonant with the optical cavities will pass through the optical cavities, and can be detected. To this end, the apparatus 1 comprises a light outlet 205 arranged to receive light from at one or more of the optical cavities 203. In this way, light of particular frequencies can be sampled. By matching the resonant frequency of the optical cavity to a target absorption feature of the target gas species (e.g. an absorption peak), the absorption of light in the test chamber 200 at that particular wavelength (or a band around that wavelength) can be detected. If the detected absorption matches that expected for the target gas, the presence of the target gas can be deduced.

In the illustrated embodiment, the light inlet 204 is located in the first part 101, and the light outlet 205 is located in the second part 102. Alternatively, both light inlet 204 and light outlet 205 may be located in the same part 101, 102. Indeed, the light inlet 204 and light outlet 205 may be co-located. That is light may be coupled into the test chamber 200 through the same component as light is received from the test chamber 200. A light inlet 204 and/or light outlet 205 may be included on both of the first part 101 and second part 102. Thus, for example, light may be detected from both sides of the apparatus 1. The light inlet 204 and light outlet 205 act as light passages through the first and second parts 101, 102. The light inlet/outlet 204, 205 (and similarly inlet/outlet 304/305 discussed below) may be a section of the first or second part 101, 102. For example, where the first and/or second parts 101, 102 are formed of silicon, and the apparatus 1 is designed to detect absorption features in the NIR range, incident NIR light may be able to pass through the silicon to enter the chamber 202. For VIS wavelength ranges, the first and or second part 101, 102 may be formed of materials such as silicon nitride, silicon carbide, quarts, or sapphire, which are transparent to VIS light. Alternatively, the light inlet/outlets may be formed by a different, suitably transparent, material to the rest of the first or second part 101, 102. In either case, the light inlet/outlet 204, 205 may comprise an anti-reflection coating, as shown in the illustrated example.

As discussed above, in conventional on-chip optical systems the absorption length is around 10-20 cm, and the achievable change in laser intensity due to methane absorption at 1650 nm is only 5ppm. This equates to a required dynamic range of more than 50 dB, which is unrealistic in any practical system.

The absorption signal can be enhanced by increasing the optical path length $l$. This can be done by increasing the distance between light source and detector, multipass spectroscopic absorption cells, or cavities such as Fabry-Perot (FP) cavities. The distance of light source to detector can be increased but this makes it impossible to keep a compact form factor. Multipass cells can let a light beam reflect between two mirrors for a couple of dozen times. For example, a standard mulitpass cell is a Heriott cell, which is typically 50 cm long and allows up to 50 m absorption length. The size and stability issues allow their operation almost only in laboratory environments and they are not suitable for online chip-based sensing applications.

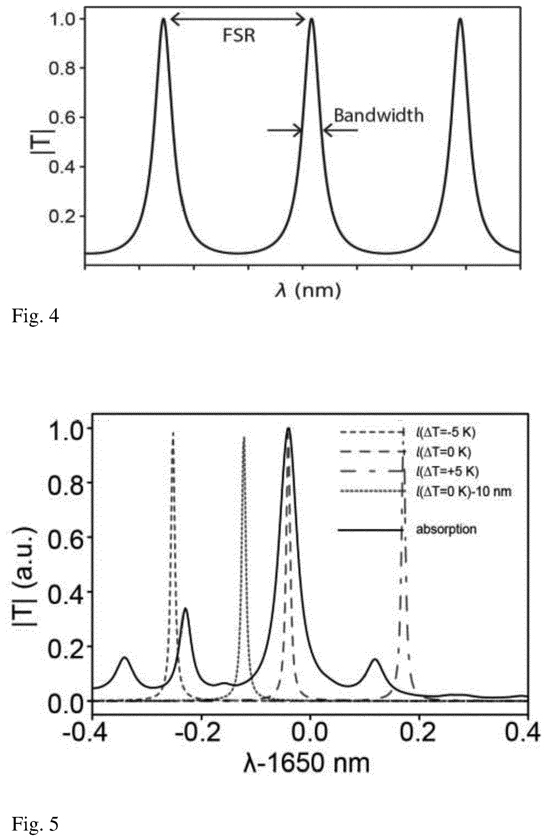

The best performing device to enhance the optical depth in any system is a cavity. For gas sensing in particular, an open-access Fabry-Perot (F-P) cavity has favourable features. Here, two mirrors form a resonator in which an optical light beam can circulate after entering the cavity. The number of round-trips a photon makes before leaving the cavity is proportional to the finesse ($\mathscr{F}$), and the effective absorption length becomes $l_{\text{eff}} = \mathscr{F} l/\pi$. The Finesse is simply the free spectral range (FSR) over the bandwidth of the cavity. Fig. 4 illustrates the mode spectra for a F-P optical cavity. Each time the length is equal to an integer multiple of the probing laser frequency, a resonance occurs. The free spectral range (FSR) depends on the length of the cavity as FSR= $c/2 n l$, with c the speed of light, $l$ the mirror spacing and $n$ the refractive index of the atmosphere inside the F-P cavity.

Thus particular examples of the gas-sensing apparatus 1 use F-P optical cavities 203. By using micromirrors to form the F-P optical cavities 203, the apparatus 1 can be highly miniaturized, and can employ on-chip F-P cavities 203. In particular, high-finesse optical micro-cavities may be fabricated into semiconductor (e.g. Si) wafers forming the first part 101 and second part 102. This concept allows use of semiconductor microfabrication processes to build monolithic optical sensors on a silicon wafer platform which are extremely compact, stable, and cheap. This approach can improve the sensitivity limit of compact optical gas sensors by up to three orders of magnitude.

The optical cavities 203 may have (physical) lengths in the range from 1 $\mu$m to 1000 $\mu$m, or from 5 $\mu$m to 500 $\mu$m, or from 150 $\mu$m to 350 $\mu$m. The optical cavities may have a volume of ~50 fL to ~400 pL. The optical cavitites may have an optical finesse at least 10,000, or at least 50,000, or at least 100,000 or at least 250,000, or at least 500,000, or more.

The theoretical shot noise detection limit $(\alpha)_{min}$ of an example of apparatus 1 can be given by:

$$(\alpha)_{min} = \frac{\pi}{2\mathscr{F}l} \sqrt{\frac{2e}{\eta P}} = 1 \times 10^{-10} \left(\frac{1}{\text{cm}\sqrt{\text{Hz}}}\right)$$

with the elementary charge $e$, assuming a Finesse $F$ = 250,000, cavity length $l$ = 200 $\mu$m, detector responsivity $\eta$ = 0.9/A and a light intensity of $P$ = 1 mW hitting the photo detector. This is more than four orders of magnitude smaller, for a one second integration time, than compared to the natural atmospheric abundance of methane:

$$(\alpha)_{\text{nat.abund}} \approx 1 \times 10^{-6} \left(\frac{1}{\text{cm}}\right).$$

Whilst this high fundamental sensitivity limit may be hard to reach in any real device, the extremely small form factor and compactness of the apparatus 1 is key in realising high sensitivities. The size in general and semiconductor fabrication techniques employed herein allow fabrication of mechanically and thermally stable optical systems.

Considering thermal stability, the thermal expansion of a 200 μm long all silicon micro-cavity 203 under a 1 Kelvin temperature change is only 0.5 nm. This is on the sub-percent level compared to the cavity resonance wavelength. In contrast, for a 20 cm long macroscopic cavity (as in conventional systems), the thermal expansion would be at least 500 nm. This equates to ~ 30 % of the resonance wavelength. Thus the optical micro-cavities 203 of the present apparatus 1 provide far superior operation regarding thermal stability compared to conventional systems. As a result, the sensitivity of the absorption measurements is much improved. Moreover, the small variation with temperature allows thermal control to be used to fine tune the optical cavities 203. As an example, Fig. 5 shows how the thermal expansion of a Si cavity with a length of 200 μm changes the resonance condition of the cavity. The absorption coefficient of methane around 1650 nm is also shown. As can be seen from this figure, small changes in temperature can be used to precisely control the resonance condition of the cavity 203. Indeed, a change of less than 5° K is needed to tune the cavity across the absorption features of methane. Such fine tuning of the cavities is discussed further below.

The small size of the optical cavities 203 also improves the mechanical stability of the detection. Consider as an example a laser beam with a pointing error of 1 degree. The offset of the beam from the optical axis on a conventional cavity of length of 20 cm will be ~ 4 mm, which corresponds to multiple times the beam waist in such a long cavity. For a 200 μm long microcavity this would only be 0.4 μm, which is less than five percent of the beam waist in the cavity. Therefore, the cavities 203 of apparatus 1 are superior to conventional gas detectors with respect to mechanical stability.

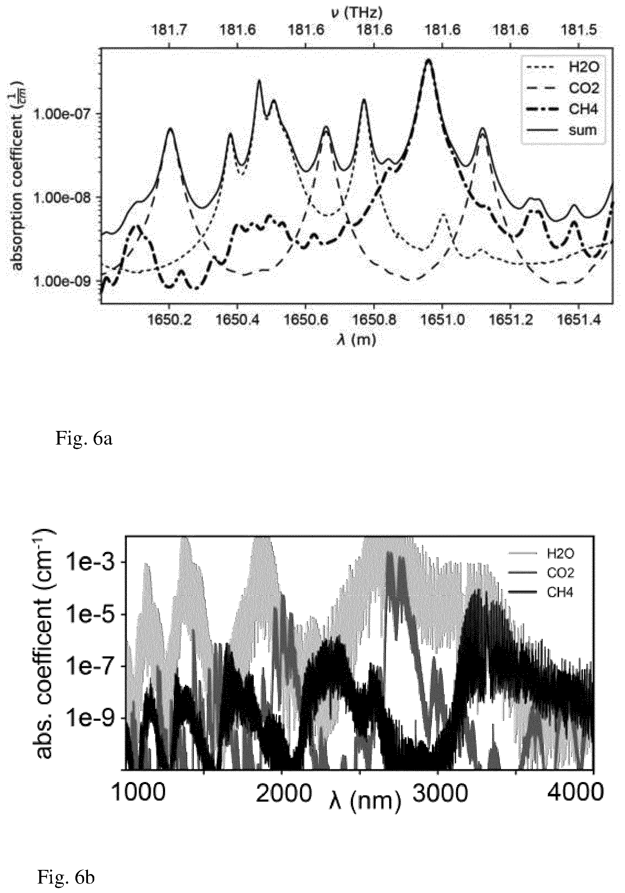

The optical cavities 203 may be configured to have resonances in the visible (VIS) to near-infrared (NIR) electromagnetic ranges (from 380 nm to 3000 nm). Fundamental absorption bands of gas species, which tend to give the strongest absorption signals, tend to be in the mid-infrared (MIR) range. The natural choice for a gas-sensor would therefore be to target the MIR absorption bands. However, MIR light sources and detectors tend to be expensive, prohibiting their use for large-scale deployment. However, the improved sensitivity of the present gas-sensing apparatus 1 allows higher absorption overtones in the VIS-NIR range to be targeted. Fig. 6a shows absorption coefficients for methane, carbon dioxide, and water in the NIR range. A clear window at which methane can be detected is at 1650nm. Fig. 6b shows absorption coefficients of the same gases across a larger wavelength range.

Advantageously, this range coincides with the optical telecoms wavelength domain. As a result, low cost components and highly sophisticated manufacturing techniques are available for this domain. Such components are used in consumer handheld devices, and so are extremely power efficient and compact. Using such components, fabrication of low-cost, portable gas-sensing devices can be achieved.

The discussion above of the benefits and arrangement of optical cavities 103 in the test chamber applies equally to the reference chamber 300, for examples comprising a reference chamber 300. As illustrated in Fig. 1, the reference chamber 300 also comprises one or more pairs of micromirrors 301, 302. Micromirrors 301, 302 are formed similarly to micromirrors 201, 202. The pairs of micromirrors 301, 302 each form a reference optical cavity 303. In the illustrated embodiment, a light inlet 304 is arranged to couple light into one or more of the reference optical cavities 303. A light outlet 305 is arranged to receive light from one or more of the reference optical cavities 103. As with light inlet/outlet 204, 205, light inlet 304 and/or light outlet 305 may be located on either or both of the first and second parts 101, 102. Light inlet 304 and light outlet 305 may be combined. The features described above in relation to the light inlet/outlet 204, 205 apply equally to the light inlet/outlet 304, 305.

Due to the small size of the apparatus 1 made possible by the use of micromirrors and microcavities, the reference chamber 300 is physically very close to the test chamber 200. Therefore, any excess noise can largely be cancelled out since detection and reference optical paths are subject to common noise sources. Such noise may be mechanical noise, laser noise and electronic noise.

The reference chamber 300 may comprise a corresponding pair of micromirrors 301, 302 for each pair of micromirrors 201, 202 in the test chamber. For example, where multiple optical cavities 203 are used to detect a single target gas, the reference chamber 300 may comprise a corresponding set of reference optical cavities 303 to provide reference signals for each test optical cavity 203. Where the test chamber 200 comprises multiple optical cavities 203 for detecting multiple target gases, the reference chamber 300 may also comprise multiple optical cavities 203 for providing reference signals for those target gases. In such cases, the reference gases held in the reference chamber 300 may comprise each of the target gases. Alternatively or additionally, the apparatus 1 may comprise multiple reference chambers 300, each reference chamber comprising one or more pairs of micromirrors 301, 302 forming reference optical cavities 303. In such cases, even where a single test chamber 200 is used to detect multiple target gases, each reference chamber 300 may be arranged to provide a reference signal for only one of, or a subset of, the target gases. In such cases, each reference chamber 300 may be filled with reference gases comprising the respective target gas/es for that reference chamber 300.

Pairs of micromirrors 301, 303 of the reference chamber 300 corresponding to pairs of micromirrors 201, 202 of the test chamber 200 may be arranged to form optical cavities 303 with resonant frequencies that are substantially equal to their counterpart in the test chamber 200. Substantially equal may mean any difference between the reference and test resonant frequencies is less than the linewidth of the absorption feature they are arranged to measure. In practice, this may be a difference of 20 GHz or less, or preferably 10 GHz or less, for absorption features in the NIR-VIS range.

In some examples, the resonant frequency of the reference optical cavity 303 may be different to the corresponding test optical cavity 203 it provides a reference signal for. For example, a different gas species may be used to provide the reference. Alternatively, different absorption features of the same gas species may be used to provide the test and reference signals.

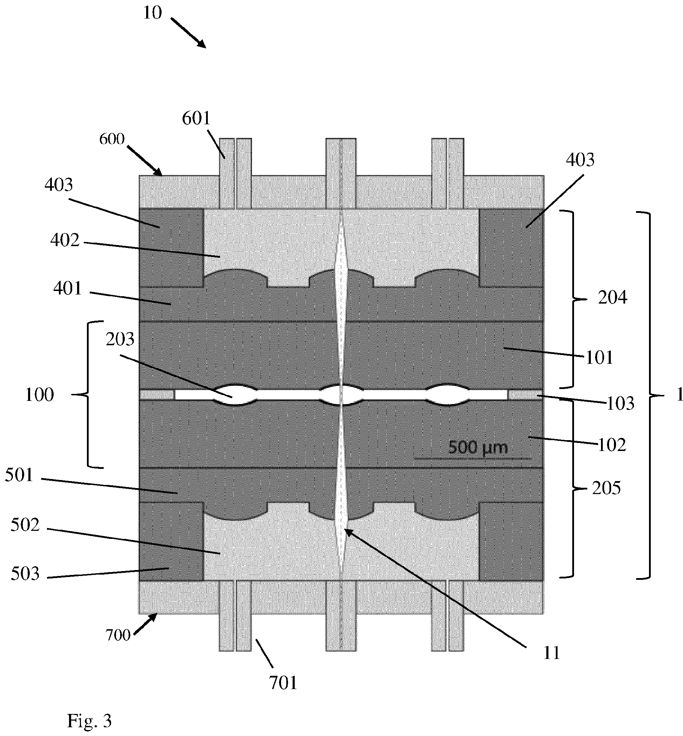

The gas-sensing apparatus 1 may be combined with a light source and light detector to form a gas detector. Fig. 3 illustrates an example of such a gas detector 10 with a light beam 11 passing through an active channel. Gas detector 10 comprises a gas-sensing apparatus 1 with body 100 and test chamber 200. Although not illustrated, the gas-sensing apparatus 1 may further comprise a reference chamber 300.

The gas detector 10 comprises a light emitting system 600 arranged to transmit light into the light inlet 204 of the gas-sensing apparatus 1. Where present, the light emitting system also transmits light into the reference inlet 304. In this example, the light emitting system 600 comprises an array of optical fibres 601, coupling light from a light source (not illustrated) into the light inlet 204. The light source may for example be a laser, LED, or any other light source emitting light wavelengths matching the resonance frequencies of the optical cavities 203 (and reference optical cavities 303, where present). The light source may be locked onto one or more cavities 203, 303, for example using Pound-Drever-Hall, dither or side of fringe locking techniques. The light source may be common to multiple gas detectors 10. The number of optical fibres 601 coupled to the device 10 matches the number of optical cavities 203 (and optionally reference optical cavities 303) to be probed. This may be all of the optical cavities 203, or only one or a subset of the optical cavities 203, as discussed further below.

In the illustrated example, the light inlet 204 comprises layers 401, 402 in addition to the passage through the first part 101 discussed above. Layer 401 is a lens array comprising a plurality of lenses (or microlenses). Each lens is configured to focus received light with a respective optical cavity 203 of the test chamber 200. The lenses of the lens array 401 may be silicon lenses. An index matching layer 402 couples light between each optical fibre 601 and layer 401. The refractive index of the index matching layer 402 matches that of the optical fibres 601. Light coming from an optical fibre 601 will expand in the index matching layer 402, and then be focused by a lens of layer 402 into a respective cavity 203. Index matching layer 402 may be a multi-layered structure. An alignment structure 403 connects and aligns the light emitting system 600 to layer 401. Alignment structure 403 controls the distance and lateral position of and between system 600 (comprising the layer of optical fibre inputs 601 in this example) and layer 403. Alignment structure 403 may be formed for a semiconductor such as silicon.

Light is coupled into the apparatus 1 from one or more of the optical fibres 601, and focused into a respective ones of the optical cavities 203 (and similarly 303). Light escaping the optical cavities 203 is then received by the light outlet 205. In the illustrated embodiment, the light outlet comprises layers 501, 502, and 503 as well as the optical passage through the second part 102 as described above. Layer 503 is an alignment structure, similar to alignment structure 403. Layer 502 is an index matching layer, similar to index matching layer 502. Layer 501 is a lens array, similar to lens array 401. Lens array 501 comprise a respective lens (or microlens) for each optical cavity 203 (and optionally each reference optical cavity 303). The lenses of lens array 501 receive light from their respective optical cavities 201, and direct it into a light detecting system 700.

Light detecting system 700 is arranged to receive light from the light outlet 204 of the gas-sensing apparatus 1. The light detecting system 700 is configured to generate a signal representative of the intensity of the received light. In the illustrated example, the light detecting system 700 comprises a plurality of optical fibres 701. Each optical fibre 701 corresponds to one of the optical cavities 203 from which measurements are to be taken. This may be all the optical cavities 203, one of the optical cavities 203, or a subset of the optical cavities 203. The lenses of the lens array 701 focus light into a respective optical fibre 701 through the index matching layer 502. The refractive index of the index matching layer 502 matches that of the optical fibres 701. The optical fibres 701 transmit the light to an optical detector (not illustrated), such as a photodiode. The optical detector is configured to determine the intensity of the received light, and hence determine the amount of light absorbed by gases in the test chamber 200 (and optionally the reference chamber 300).

In the example described above, the light source and light detector were described as part of the gas detector 10, connected to the gas-sensing apparatus 1 within gas detector 10 by optical fibres 601, 701. In some examples of gas detector 10, the light emitting system 600 and/or light detecting system 700 may be removably coupled to the gas-sensing apparatus 1. For example, the apparatus 1 may comprise ports configured to receive one or more optical fibres 601, 701, and to couple light from those optical fibres 601, 701 into one or more optical cavities 203 (and/or reference cavities 303).

Whether or not lens array 501, and/or lens array 601 are used may depend on the shape of the micromirrors 201, 202. If both micromirrors 201, 202 of a pair are concave, lens arrays 501, 502 may be useful for correctly coupling light into the respective cavity 203. If one or both micromirrors 201, 202 of a pair is planar, the lens arrays 501, 601 may be omitted.

The optical fibres 601, 701 discussed above allow a remote light source/light detector to be connected to the gas-sensing apparatus 1. In some examples, however, the light source and/or light detector may be directly incorporated into the gas-sensing apparatus 1. For example, the light source and/or light detector may be attached to one of the first part 101 and second part 102. Such a light source may comprise a laser diode, for an example a vertical-cavity surface-emitting laser (VCSEL), a difference freqeuncy generation (DFG) laser diode, or a distributed feedback (DFB) laser diode. Such a light detector may be a photodiode. By incorporating the light source and/or light detector into the gas-sensing apparatus 1, a complete, compact gas detector 10 can be produced.

The gas detector 10 may further comprise a gas detection system. The gas detection system is configured to receive a signal representative of the intensity of the light from the light detection system, and to determine a proportion of light from the light source absorbed in the gas-sensing apparatus. The gas detection system may then determine whether one or more target gases are present in the test chamber based on the proportion of light absorbed in the gas-sensing apparatus. This may include determining a concentration of the one or more target gases present in the chamber. The gas detector may comprise a processor configured to receive the signal from the light detection system, determine the proportion of light absorbed, and output the determination of presence of target gases. Such a processor may be incorporated into the gas-sensing apparatus 1. The gas detection system may be a computer or embedded system to which the gas detector 10 is connected (by either a wired or wireless connection).

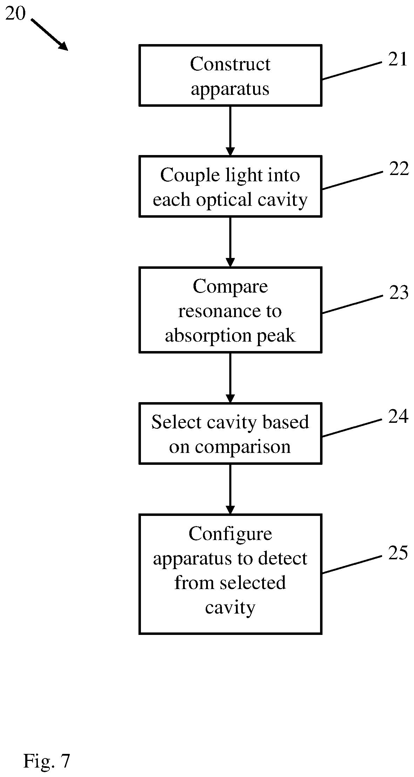

As discussed above, the gas-sensing apparatus 1 may be fabricated with a multiple optical cavities 203 and/or reference cavities 303 targeting the same target gas. Each of these cavities 203, 303 may have a slightly different resonant frequency, for example due to manufacturing tolerances. It may therefore be advantageous to select one or more of the plurality of cavities 203 that best match the intended absorption feature of the target gas/es. To this end, the gas-sensing apparatus 1 may be calibrated before use to select one or more of the optical cavities 203 and/or reference optical cavities 303. This may comprise measuring the frequency of light output from each optical cavity 202 (and/or reference optical cavity 303), and determining which optical cavities 202, 303 have a resonance closest in frequency to the target absorption feature. Such a method is discussed below in relation to Fig. 7.

As a result, it may not be necessary to detect light from all the optical cavities 203 after the gas-sensing apparatus 1 has been calibrated. Thus in some examples, the light inlet 204 is arranged to couple light into only a subset of the optical cavities 203 (and similarly for reference optical cavities 203). The subset of optical cavities 203 comprises one or more optical cavities selected based on one or more target gas species to be detected by the gas-sensing apparatus 1. In other words, the subset of optical cavities 203 are those selected in a calibration process to best match the target absorption features of the target gases. Similarly, light outlet 205 may be arranged to receive light from only the subset of the optical cavities. Alternatively, light may still be coupled into all optical cavities 203, but the light detection system or gas detection system may disregard measurements from the non-selected optical cavities 203.

As discussed above, the resonant frequency of the optical cavities 203, 303 is set on a rough scale by the spacing between the surface of the first and second parts 101, 102 on which the pairs of micromirrors 201, 202, 301, 302 are formed. In the illustrated examples, this spacing is set by the thickness of the spacing structure 103. Smaller variations in resonant frequency between the optical cavities 203, 303 can be created by the form of micromirrors 201, 202, 301, 302. The best optical cavities 203, 303 for the target gas can be selected using a calibration process, as described above. However, it would still be advantageous to allow for fine-scale tuning of the resonance frequencies of the optical cavities 203, 303, after calibration, and even during use of the gas-sensing apparatus 1.

To this end, some examples of the gas-sensing apparatus 1 (and/or gas detector 10) comprise an optical cavity tuning system 104. The tuning system 104 allows fine-tuning of the resonance frequencies of the optical cavities 203 (and/or reference optical cavities 303). In particular, the tuning system 104 may be arranged to cause small variations in the spacing between pairs of micromirrors 201, 202 and/or reference micromirrors 301, 302. Adjusting the spacing in turn adjusts the resonant frequency of the optical cavities 203, 303. In some examples, the spacing between the first part 101 and second part 102 may be adjusted, tuning all optical cavities 203 (and, where present reference optical cavities 303) simultaneously. Alternatively, the tuning system 104 may be configured to tune individual optical cavities 203, 303 by altering the spacing or shape of the micromirrors 201, 202, 301, 302 forming those cavities 203, 303.

In the example illustrated in Fig. 1, the tuning system 104 is shown applying a signal to the spacing structure 103. The signal is arranged to control the thickness of the spacing structure 103 (i.e. the thickness in the direction parallel to the spacing between the first part 101 and second part 102). Varying the thickness of the spacing structure 103 alters the spacing between pairs of micromirrors 201, 202, 301, 302, and thus adjusts the resonant frequency of the optical cavities 203, 303.

In some examples, the optical cavity tuning system 104 is arranged to change the temperature of the spacing structure 103 (or generally the body 100, to cause a thermal expansion/contraction of the material of the body 100). The tuning system 104 may be or comprise a heater/cooler attached to the spacing structure 103/body 100. The tuning system 104 may further comprise a temperature controller, arranged to monitor the temperature of the spacing structure 103/body 100 and apply a signal to the heater/cooler accordingly. Alternatively, the tuning system 104 may be configured to apply an electrical signal to the spacing structure 103/ body 100 which causes the spacing structure 103/body 100 to change temperature (e.g. by resistive heating). Similarly, the tuning system 104 may apply heat or a signal directly to one or both of a pair of micromirrors 201, 202, 301, 302 to individually tune the optical cavity 203, 303 they form.

Additionally or alternatively, the optical cavity tuning system 104 may be configured to apply a piezoelectric-controlling voltage signal to the spacing structure 103 and/or body 100. In such examples, the spacing structure 103/body 100, or parts thereof, comprises a piezoelectric active material. The piezoelectric material is arranged such that, upon application of an electric field, the piezoelectric material causes the spacing between the first part 101 and second part 102 to increase or decrease. The maximum spacing change the optical cavity tuning system 104 can apply may be in the range from 10 pm to 500pm, or 10 pm to 100 pm, or 25 pm to 75 pm. For example, the thickness of the spacing structure 103 may be controlled in this way. The tuning system 104 may comprise electrodes attached to the piezoelectric material, and an electrical signal generator configured to apply a voltage to the electrodes in order to control the piezoelectric active material. In an example, one or more first electrodes are attached to or incorporated into a first surface of the spacing structure 103 facing the first part 101. One or more second electrodes are attached to or incorporated into a second surface of the spacing structure 103 facing the second part 102. The first and/or second surfaces may be substantially completely covered by respective first and second electrodes. The spacing structure itself is formed of a weakly piezoelectric material. The optical cavity tuning system 104 is configured to apply a voltage between the first electrodes and second electrodes. The spacing structure 103 forms a capacitor and with an electric field built up between the first and second electrodes the piezoelectric crystal expands or contracts, causing the thickness of the spacing structure to expand or contract, and hence altering the distance between the first part 101 and second part 102. An example for a spacing structure 103 material is aluminium nitride (A1N) with a dielectric coefficient d33=5 pC/N. If a voltage of 10 Volts is applied onto such a spacing structure 103, it expands or contracts approximately 50 pm, which is sufficient to scan over an absorption feature or even to lock the cavity to the light input laser. The applied voltage signal can be D.C, or A.C up to a frequency of hundreds of kilo Hertz. Other suitable piezoelectric materials include Lithium Niobate, Zinc Oxide, Quartz or any other suitable material.

Similarly, one or more of optical cavities 203, 303 may be individually tuned by applying an electric signal to a piezoelectric active material associated with the respective pair of micromirrors 201, 202, 301, 302. For example at least one of the pair of micromirrors 201, 202, 301, 302 may be formed on or from a piezoelectric active material.

The optical cavity tuning system 104 may be used to tune one or more of the optical cavities 203 and/or reference cavities 303 during an absorption measurement. In this way the gas-sensing apparatus can scan across a range of frequencies during the measurement, rather than taking measurements only at single frequency points. This may allow an absorption feature of the target gas/es to be more accurately identified.

Alternatively or additionally, the tuning system 104 may be used to thermally stabilise the optical cavities, reducing the thermal noise in the absorption measurements. As discussed above in relation to Fig. 5, thermal expansion of the body 100 can detune the optical cavities 203, 303 from the frequency of their intended absorption feature. The optical cavity tuning system 104 can be used to correct for such detuning. In such examples, the optical cavity tuning system 104 is arranged to monitor the resonant frequencies of one or more the optical cavities 203, 303, and to alter the resonant frequency of one or more of the optical cavities based on the monitored resonant frequencies. For example, the tuning system 104 may receive a signal representative of the current resonant frequency of an optical cavity 203, 303 (e.g. as measured by a detection system), or representative of a change in the resonant frequency. The tuning system 104 can then tune the optical cavity 203, 303 based on the received signal. Thus a feedback loop may be formed.

The optical cavity tuning system 104 can be used to compensate for slow drifts like temperature and/or to directly lock a cavity 203, 303 to the laser (used as part of the light input system). For this the control has to be fast, this could be done by a fast piezoelectric shift or by temperature tuning. In the latter case of fast tuning the cavity 203, 303 would be locked to the laser and not the other way around. This is preferable since many cavities 203, 303 could be locked to one common laser. A feedback loop for locking may use a locking error signal which is fed into the optical cavity tuning system 104 instead of the laser to lock and stabelize the system. Alternatively, for slow control a temperature or piezolectric signal can be used to control the cavity 203, 303 with respect to a laser or absorption feature in the refernce chamber. For example if a laser is calibrated the slow tuning can be used to match the cavity resonance frequency to the laser or vice versa.

Monitoring the resonant frequency in some examples comprises monitoring one or more of the reference cavities 303 in the reference chamber. The resonant frequency of these reference cavities 303 may then be measured with respect to a calibrated light source. Alternatively, the absorption of one or more reference gases in the reference chamber 300 may be monitored, from which a change in the resonant frequency of one or more reference cavities 303 can be calculated.

Thus the gas-sensing apparatus 1 allows the precision of microfabrication processes (especially when formed of semiconductor wafers) to be combined with high precision tuning of the optical cavities. Temperature tuning can provide a control of the resonant wavelegnth of ~pm/mK precision. Piezoelectric tuning can provide ~pm/V precision. This allows for very high sensitivity absorption measurements, in a small form-factor device.

Fig. 7 illustrates a method 20 of providing a gas-sensing apparatus 1 for use in detecting presence of a target gas species.

Method 20 starts at step 21, at which a gas-sensing apparatus 1 is constructed by forming a test chamber 200 between a first part 101 and a second part 102. The test chamber comprises a plurality of pairs of micromirrors 201, 202, one of each pair of micromirrors 201, 202 being disposed on the first part 101 and the other of each pair of micromirrors 201, 202 being disposed on the second part 102. Each pair of micromirrors 201, 202 forms a respective optical cavity 203. The gas-sensing apparatus may generally be constructed to have any of the features of the apparatus 1 or device 10 discussed above, such as a reference chamber 300.

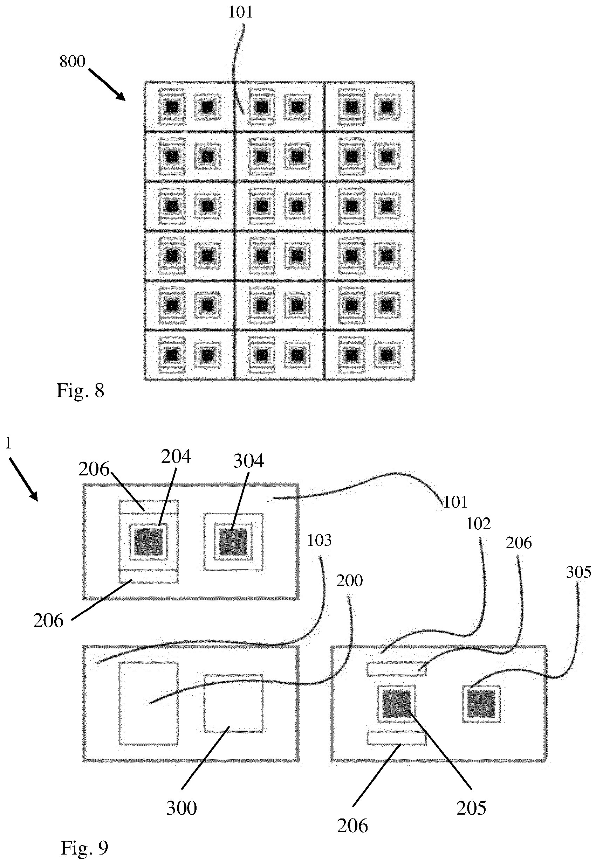

As noted above, the gas-sensing apparatus 1 may advantageously be constructed using semiconductor (e.g. Si) wafers. Figs. 8 and 9 illustrate an example process of forming a plurality of gas-sensing apparatus 1 from semiconductor wafers 800. In this process, each of the first part 101, second part 102, and spacing structure 103 are formed by optical lithography and reactive ion etching on respective wafers 800. Fig. 8 shows an example of a first wafer 800 on which a plurality of first parts have been etched.

Micromirrors 201, 202 (and optionally reference micromirrors 301, 302) are formed on the wafers 800 used for the first and second parts 101, 102. These wafers may be coated with high reflectivity coatings to form the micromirrors. Anti-reflective coatings may be used to form the light inlet/outlets 204, 205, to facilitate low loss transmission through the first and second parts 101, 102. Gas inlets 206 are also formed on wafers 800 forming the first and/or second parts 101, 102 by the lithography and etching process. All three wafers 800 are then bonded together under a controlled atmosphere. The bonding process is gas tight. The controlled atmosphere may be used to fill and seal any reference chamber 300 with the reference gases. For example, the bonding is performed under an atmosphere of inert gas such as nitrogen which contains a reference gas such as $CH_4$, $CO_2$ or Co. After bonding the wafers 800, the wafer sandwich is diced into smaller dies. Fig. 9 illustrates separately the three layers 101, 102, 103 of an individual die forming a gas-sensing apparatus 1.

After the apparatus 1 has been constructed, the method 20 proceeds to step 22. At step 22, light is coupled into each optical cavity 203 to determine a resonant frequency of each optical cavity 203. The light may be coupled into each optical cavity 203 in turn, or all cavities 203 simultaneously, depending on the detection arrangement. The light may be provided by any light source, such as those discussed above in relation to light emitting system 600. Light exiting the optical cavities 203 after absorption are detected by a detection system configured to determine the frequency of received light. For example the detection system may compared the received light to a calibrated light source of known frequency. Any reference cavities 303 may similarly be tested.

The method 20 then proceeds to step 23, at which the determined resonance frequencies are compared to the frequency of a target absorption feature of the target gas species, such as a peak in the absorption coefficient. For example, in the case of methane the target absorption peak may be the absorption peak around 1650 nm.

The method 20 then proceeds to step 24, at which one (or more) of the plurality of optical cavities 203 is selected based on the comparison of resonance frequencies to the frequency of the absorption peak. For example the cavity/ies with a resonance frequency closest to the frequency of the target absorption feature may be selected.

The method 20 then proceeds to step 25, at which the gas-sensing apparatus 1 is configured to detect light from the selected optical cavity. For example, this may comprise connecting a light emitting system 600 and/or light detection system 700 such that only the selected cavity/ies 203 are sampled. Alternatively measurements may be taken from all cavities 203, but measurements from non-selected cavities 203 are discarded. Alternatively, input and output channels for non-selected cavities 203 may be blocked.

The method 20 may be repeated for each target gas the apparatus 1 is intended to detect. Similarly it may be repeated for each reference gas the reference optical cavities 303 are intended to detect. In some examples, the selection process of steps 22-25 may not be needed, for example where tuning system 104 can be relied upon to provide sufficient control over resonance frequencies.

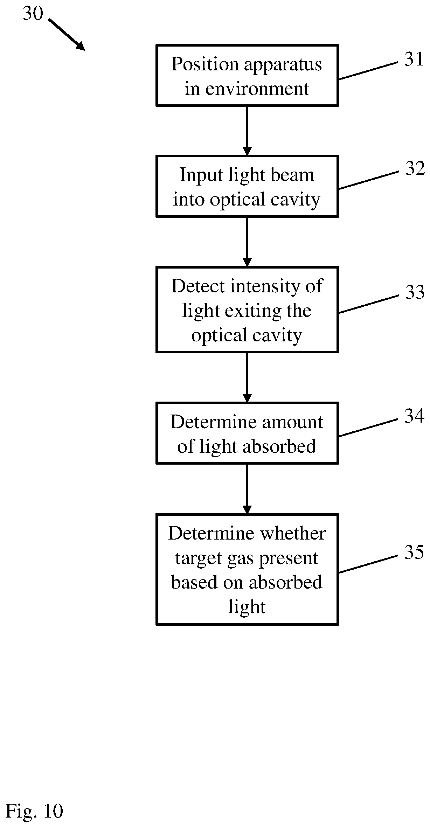

Fig. 10 illustrates a method 30 of detecting the presence of a target gas species in an environment, using a gas-sensing apparatus 1 (or gas sensing device 10).

Method 30 starts at step 31, at which a gas-sensing apparatus 1 is positioned in the environment such that gasses from the environment enter the test chamber 200 of the apparatus 1 via the gas inlet 206. The apparatus 1 may comprise any of the features discussed above.

At step 32, a light beam is input into one or more optical cavities 203 of the gas-sensing apparatus 1. For example a light emitting system 600 may be used to couple light into the apparatus 1.

At step 33, the intensity of light exiting the optical cavity/cavities is detected. For example, a light detection system 700 may be used to receive the light, and determine its intensity.

At step 34, an amount of light absorbed in the optical cavity (i.e. absorbed during passage through the optical cavity 203, due to the gas in test chamber 200) is determined. For example the measured intensity may be compared to an initial intensity of the light input into the optical cavity 203 to deduce the amount absorbed in the optical cavity 203.

At step 35, it is determined whether the target gas species is present based on the amount of light absorbed in the optical cavity 203 or optical cavities 203. This may comprise determining the concentration of the target gas present in the test chamber 200.

A variety of detections schemes may be used to detect and characterize the light passing through the optical cavities 203. For example, where the apparatus 1 comprises a reference chamber with one or more reference optical cavities 303 corresponding to the test optical cavity/ies 203, a balanced detection scheme may be used. Light may be coupled into both the optical cavity/ies 203 and the corresponding reference optical cavity/ies 303. Both are detected, and are compared (e.g. subtracted or divided) to reduce the impact of noise on the received signal.

Alternatively or additionally, wavelength or frequency modulation spectroscopy techniques may be employed. In such cases, the resonance of the cavity 203 is modulated rapidly over the absorption feature (using tuning system 104). Here, rapidly means faster than noise in the system. Only light that shows the same modulation is then detected (e.g. using lock-in detection techniques), reducing the amount of noise in the detected signal.

Alternatively or additionally, in-situ heterodyne detection schemes may be employed. In such cases a laser is modulated with a frequency comparable to the bandwidth of the target molecular absorption feature, sent into the one or more cavities 203, and interfered on a photodetector. Alternatively or additionally, cavity ring down spectroscopy may be employed.

Although fig. 10 describes detecting target gases based on the amount of light absorbed, in general any suitable property (or combination of properties) of the detected light may be analysed to determine the presence of a target gas. For example, the detected light may be analysed to detect at least one of: a shift in a resonance frequency of the optical cavity; a change in a ring-down time of the optical cavity; and a change in a linewidth of the optical cavity. The presence of the gas species may then be determined based on at least one of: the amount of light absorbed in the optical cavity; a magnitude of a resonance wavelength shift; a magnitude of the change in the ring-down time; and a magnitude of the change in linewidth.

Such detection techniques reduce the laser, mechanical, and/or thermal noise in the detected signal, and so improve the sensitivity of the gas-sensing apparatus 1.

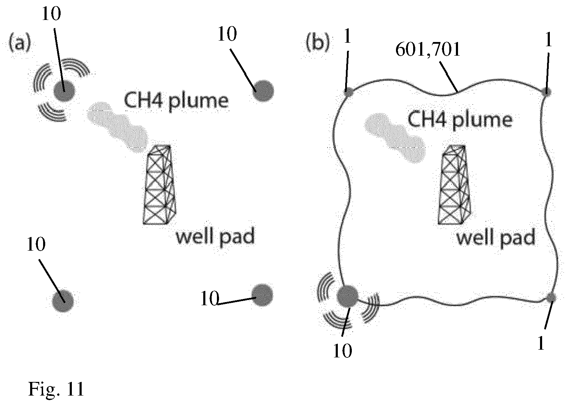

Figs. 11(a) and (b) illustrate examples of the method 30 put into practice. These figure is a schematic representation of a number of gas-sensing apparatus 1 used to monitor natural gas leaks on a well pad. In Fig. 11(a), multiple individual gas detectors 10 monitor and observe natural gas plumes. The low cost and portability of the present devices mean multiple devices 10 can be easily positioned around an individual well pad or alongside a pipeline. In Fig. 11(b), multiple gas-sensing apparatus 1 are arrayed around the well pad. The gas-sensing apparatus 1 are connected by a fibre optics to a gas detector 10. The gas detector 10 acts as a base station for the collection of apparatus 1. The network of fibre optics allows light emitting system 600 and/or light detection system 700 of the detector 10 to be shared with the multiple apparatus 1. The gas detector 10 can also transmit the results (i.e. detection or no detection of the target gas/concentration of the target gas) to a remote server on behalf of all the apparatus 1. This saves reduces number of electronics and laser sources needed, further saving on costs.

The following clauses define further statements of invention:

1. A gas-sensing apparatus comprising:

    a test chamber formed in a body, the test chamber comprising a pair of micromirrors, one of the pair of micromirrors being disposed on the first surface of the body and the other of the pair of micromirrors being disposed on the second surface of the body, wherein the pair of micromirrors forms an optical cavity;
    a light inlet arranged to couple light into the optical cavity;
    light outlets arranged to receive light from the optical cavity; and
    a gas inlet configured to allow gas from outside of the apparatus to enter the test chamber.

2. The apparatus of clause 1, wherein the body comprises a first part and a second part, the test chamber being formed between the first part and the second part, and wherein the first surface is a surface of the first part, and the second surface is a surface of the second part.

3. The apparatus of clause 2, wherein the first part is a first substrate and the second part is a second substrate, and wherein the body further comprises a spacing structure separating the first substrate and the second substrate.

4. The apparatus of any of clauses 1-3, wherein the test chamber comprises a plurality of pairs of micromirrors, one of each pair of micromirrors being disposed on the first surface and the other of each pair of micromirrors being disposed on the second surface, wherein each pair of micromirrors forms a respective optical cavity;
    wherein the light inlet is arranged to couple light into one or more of the optical cavities; and
    wherein the light outlet is arranged to receive light from one or more of the optical cavities.

5. The apparatus of clause 4, wherein the light inlet is arranged to couple light into a subset of the optical cavities, the subset of optical cavities comprising one or more optical cavities selected based on one or more target gas species to be detected by the gas-sensing apparatus.

6. The apparatus of clause 4, wherein the light outlet is arranged to receive light from a subset of the optical cavities, the subset of optical cavities comprising one or more optical cavities selected based on one or more target gas species to be detected by the gas-sensing apparatus.

7. The apparatus of any of clauses 4-6, wherein the test chamber comprises a plurality of pairs of micromirrors for each of one or more target gas species to be detected by the gas-sensing apparatus.

8. The apparatus of clause 7, wherein the test chamber comprises 2 or more, or 5 or more, or 10 or more pairs of micromirrors for each of the one or more target gas species.

9. The apparatus of any of clauses 4 to 8, wherein the gas-sensing apparatus is configured to detect 2 or more, or 5 or more, or 10 or more target gas species.

10. The apparatus of any preceding clause, further comprising a reference chamber formed in the body, the reference chamber comprising:

one or more pairs of micromirrors, each pair of micromirrors forming a reference optical cavity;
a light inlet arranged to couple light into one or more of the reference optical cavities; and
a light outlet arranged to receive light from one or more of the reference optical cavities;
wherein the reference cavity is sealed or is sealable from outside gasses.

11. The apparatus of clause 10, wherein body comprises a first part and a second part, wherein the reference chamber is formed between the first part and the second part.

12. The apparatus of clause 10 or clause 11, wherein the reference chamber is fillable with one or more reference gas species.

13. The apparatus of any of clauses 10-12, wherein the reference chamber comprises a corresponding pair of micromirrors for each pair of micromirrors in the test chamber.

14. The apparatus of clause 13, wherein the resonant frequency of corresponding pairs of micromirrors in the test chamber and reference chamber is substantially equal.

15. The apparatus of any preceding clause, further comprising an optical cavity tuning system configured to alter the resonant frequency of one or more of the optical cavities.

16. The apparatus of clause 15, wherein the optical cavity tuning system is configured to change the temperature of the micromirrors and/or the body in order to vary the resonant frequency of one or more of the optical cavities.

17. The apparatus of clause 15 or clause 16, wherein, wherein the micromirrors and/or the body comprise a piezoelectric material, and wherein the optical cavity tuning system is configured to apply a piezoelectric control signal to piezoelectric material in order to vary the resonant frequency of the one or more of the optical cavities.

18. The apparatus of any of clauses 15 to 17, wherein the optical cavity tuning system is arranged to monitor the resonant frequencies of one or more the optical cavities, and to alter the resonant frequency of one or more of the optical cavities based on the monitored resonant frequencies.

19. The apparatus of any preceding clause, wherein the optical cavity or optical cavities are configured to have resonances in the visible to near-infrared electromagnetic ranges.

20. A gas detector comprising:

the gas-sensing apparatus of any preceding clause;
a light emitting system arranged to transmit light into the light inlet of the gas-sensing apparatus; and
a light detecting system arranged to receive light from the light outlets of the gas-sensing apparatus, wherein the light detecting system is configured to generate a signal representative of the intensity of the received light.

21. The gas detector of clause 20, wherein the light emitting system comprises a light source and an optical fibre, the optical fibre arranged to couple light from the light source into the light inlet.

22. The gas detector of clause 20 or 21, wherein the light detecting system comprises one or more photodiodes and one or more optical fibres, the one or more optical fibres arranged to couple light from the light outlet onto the one or more photodiodes.

23. The gas detector of clause 20, wherein the light emitting system and/or light detecting system is incorporated into the gas-sensing apparatus.

24. The gas detector of any of clauses 20 to 23, further comprising a gas detection system, wherein the gas detection system is configured to receive the signal representative of the intensity of the light from the light detection system, and to determine a proportion of light from the light source absorbed in the gas-sensing apparatus.

25. The gas detector of clause 24, wherein the detection system is configured to determine whether one or more target gasses are present in the test chamber based on the proportion of light absorbed in the light absorbed in the gas-sensing apparatus.

26. The gas detector of clause 25, wherein the detection system is configured to determine a concentration of the one or more target gasses present in the chamber.

27. A method of detecting presence of a target gas species in an environment, the method comprising:

positioning a gas-sensing apparatus according to any of clauses 1 to 19 in the environment such that gasses from the environment enter the test chamber of the apparatus;
inputting a light beam into an optical cavity of the gas-sensing apparatus;
detecting the light exiting the optical cavity; and
analysing the detected light to determine whether the target gas species is present.

28. The method of clause 27, wherein:

analysing the detected light comprises at least one of:

determining an amount of light absorbed in the optical cavity;
detecting a shift in a resonance frequency of the optical cavity;
detecting a change in a ring-down time of the optical cavity; and
detecting a change in a linewidth of the optical cavity; and

determining whether the target gas species is present is based on at least one of:

the amount of light absorbed in the optical cavity;
a magnitude of a resonance wavelength shift;
a magnitude of the change in the ring -down time; and
a magnitude of the change in linewidth.

29. The method of clause 27 or clause 28, wherein determining whether the target gas species is present comprises determining a concentration of the target gas present in the test chamber.

30. A method of providing a gas-sensing apparatus for use in detecting presence of a target gas species, the method comprising:

constructing the gas-sensing apparatus by forming a test chamber between a first part and a second part, the test chamber comprising a plurality of pairs of micromirrors, one of each pair of micromirrors being disposed on the first part and the other of each pair of micromirrors being disposed on the second part, wherein each pair of micromirrors forms a respective optical cavity;
coupling light into each optical cavity to determine a resonant frequency of each optical cavity;
comparing the determined resonance frequencies to the frequency of an absorption peak of the target gas species;
selecting one of the plurality of optical cavities based on the comparison of resonance frequencies to the frequency of the absorption peak; and
configuring the gas-sensing apparatus to detect light from the selected optical cavity.

**Claims**

1. A gas-sensing apparatus comprising:

a test chamber formed in a body, the test chamber comprising a pair of micromirrors, one of the pair of micromirrors being disposed on the first surface of the body and the other of the pair of micromirrors being disposed on the second surface of the body, wherein the pair of micromirrors forms an optical cavity;
a light inlet arranged to couple light into the optical cavity;
light outlets arranged to receive light from the optical cavity; and
a gas inlet configured to allow gas from outside of the apparatus to enter the test chamber.

2. The apparatus of claim 1, wherein the body comprises a first part and a second part, the test chamber being formed between the first part and the second part, and wherein the first surface is a surface of the first part, and the second surface is a surface of the second part.

3. The apparatus of claim 2, wherein the first part is a first substrate and the second part is a second substrate, and wherein the body further comprises a spacing structure separating the first substrate and the second substrate.

4. The apparatus of any of claims 1-3, wherein the test chamber comprises a plurality of pairs of micromirrors, one of each pair of micromirrors being disposed on the first surface and the other of each pair of micromirrors being disposed on the second surface, wherein each pair of micromirrors forms a respective optical cavity;
wherein the light inlet is arranged to couple light into one or more of the optical cavities; and
wherein the light outlet is arranged to receive light from one or more of the optical cavities.

5. The apparatus of any preceding claim, further comprising a reference chamber formed in the body, the reference chamber comprising:

one or more pairs of micromirrors, each pair of micromirrors forming a reference optical cavity;
a light inlet arranged to couple light into one or more of the reference optical cavities; and
a light outlet arranged to receive light from one or more of the reference optical cavities;
wherein the reference cavity is sealed or is sealable from outside gasses.

6. The apparatus of claim 5, wherein the reference chamber is fillable with one or more reference gas species.

7. The apparatus of any preceding claim, further comprising an optical cavity tuning system configured to alter the resonant frequency of one or more of the optical cavities.

8. The apparatus of claim 7, wherein the optical cavity tuning system is configured to change the temperature of the micromirrors and/or the body in order to vary the resonant frequency of one or more of the optical cavities.

9. The apparatus of claim 7 or claim 8, wherein the micromirrors and/or the body comprises a piezoelectric material, and wherein the optical cavity tuning system is configured to apply a piezoelectric control signal to piezoelectric material in order to vary the resonant frequency of the one or more of the optical cavities.

10. The apparatus of any of claims 7 to 9, wherein the optical cavity tuning system is arranged to monitor the resonant frequencies of one or more the optical cavities, and to alter the resonant frequency of one or more of the optical cavities based on the monitored resonant frequencies.

11. A gas detector comprising:

the gas-sensing apparatus of any preceding claim;
a light emitting system arranged to transmit light into the light inlet of the gas-sensing apparatus; and
a light detecting system arranged to receive light from the light outlets of the gas-sensing apparatus, wherein the light detecting system is configured to generate a signal representative of the intensity of the received light.

12. The gas detector of claim 11, wherein the light emitting system comprises a light source and an optical fibre, the optical fibre arranged to couple light from the light source into the light inlet; or
wherein the light emitting system is incorporated into the gas sensing apparatus.

13. The gas detector of any of claims 11 to 12, further comprising a gas detection system, wherein the gas detection system is configured to receive the signal representative of the intensity of the light from the light detection system, and to determine a proportion of light from the light source absorbed in the gas-sensing apparatus.

14. A method of detecting presence of a target gas species in an environment, the method comprising:

positioning a gas-sensing apparatus according to any of claims 1 to 19 in the environment such that gasses from the environment enter the test chamber of the apparatus;
inputting a light beam into an optical cavity of the gas-sensing apparatus;
detecting the light exiting the optical cavity; and
analysing the detected light to determine whether the target gas species is present.

15. A method of providing a gas-sensing apparatus for use in detecting presence of a target gas species, the method comprising:

constructing the gas-sensing apparatus by forming a test chamber between a first part and a second part, the test chamber comprising a plurality of pairs of micromirrors, one of each pair of micromirrors being disposed on the first part and the other of each pair of micromirrors being disposed on the second part, wherein each pair of micromirrors forms a respective optical cavity;

coupling light into each optical cavity to determine a resonant frequency of each optical cavity;

comparing the determined resonance frequencies to the frequency of an absorption peak of the target gas species;

selecting one of the plurality of optical cavities based on the comparison of resonance frequencies to the frequency of the absorption peak; and

configuring the gas-sensing apparatus to detect light from the selected optical cavity.

Fig. 1

Fig. 2

Fig. 3

Fig. 4

Fig. 5

Fig. 6a

Fig. 6b

20

```
┌─────────────────┐
│    Construct    │────── 21
│    apparatus    │
└─────────────────┘
          │
          ▼
┌─────────────────┐
│  Couple light into  │────── 22
│  each optical cavity │
└─────────────────┘
          │
          ▼
┌─────────────────┐
│     Compare     │────── 23
│   resonance to  │
│  absorption peak │
└─────────────────┘
          │
          ▼
┌─────────────────┐
│ Select cavity based │────── 24
│   on comparison  │
└─────────────────┘
          │
          ▼
┌─────────────────┐
│    Configure    │────── 25
│ apparatus to detect │
│   from selected  │
│      cavity     │
└─────────────────┘
```

Fig. 7

801

101

Fig. 8

1

206
204
304
206
206

101
102
206
305

103
200

300
206
205

Fig. 9

30

```
┌─────────────────────┐
│  Position apparatus │ ─── 31
│    in environment   │
└─────────────────────┘
           │
           ▼
┌─────────────────────┐
│   Input light beam  │ ─── 32
│  into optical cavity│
└─────────────────────┘
           │
           ▼
┌─────────────────────┐
│   Detect intensity of│
│   light exiting the │ ─── 33
│    optical cavity   │
└─────────────────────┘
           │
           ▼
┌─────────────────────┐
│   Determine amount  │ ─── 34
│  of light absorbed  │
└─────────────────────┘
           │
           ▼
┌─────────────────────┐
│  Determine whether  │
│   target gas present│ ─── 35
│  based on absorbed  │
│        light        │
└─────────────────────┘
```

Fig. 10

Fig. 11

**EUROPEAN SEARCH REPORT**

Europäisches
Patentamt
European
Patent Office
Office européen
des brevets

Application Number

EP 21 18 3799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 7 554 673 B2 (PALO ALTO RES CT INC [US]) 30 June 2009 (2009-06-30) * figures 14, 15 * | 1-15 | INV.<br>G01N21/35<br>G01N21/03<br>G01N33/00 |
| Y | AYERDEN N P ET AL: "A highly miniaturized NDIR methane sensor", PROCEEDINGS OF SPIE; [PROCEEDINGS OF SPIE ISSN 0277-786X VOLUME 10524], SPIE, US, vol. 9888, 27 April 2016 (2016-04-27), pages 98880D-98880D, XP060071248, DOI: 10.1117/12.2225924 ISBN: 978-1-5106-1533-5 Sections 2. and 3.; * abstract; figure 1 * | 1-15 | G01N33/22<br>G01J3/26 |
| Y | AYERDEN N PELIN ET AL: "How accurate IS the Fabry-Perot approximation in high-finesse linear variable optical filters for gas absorption spectroscopy?", 2017 19TH INTERNATIONAL CONFERENCE ON SOLID-STATE SENSORS, ACTUATORS AND MICROSYSTEMS (TRANSDUCERS), IEEE, 18 June 2017 (2017-06-18), pages 2111-2114, XP033131198, DOI: 10.1109/TRANSDUCERS.2017.7994491 [retrieved on 2017-07-26] * abstract; figure 1 * | 1-15 | |
| Y | WO 96/21140 A1 (HONEYWELL INC [US]) 11 July 1996 (1996-07-11) * page 3, lines 19-20, 24-31; figure 4 * * page 4, lines 3-4 * | 7-10 | TECHNICAL FIELDS SEARCHED (IPC)<br>G01N<br>G01J |
| A | US 2020/378892 A1 (SABRY YASSER M [EG] ET AL) 3 December 2020 (2020-12-03) * the whole document * | 1-15 | |

The present search report has been drawn up for all claims

| Place of search | Date of completion of the search | Examiner |
|---|---|---|
| Munich | 24 November 2021 | Meacher, David |

EPO FORM 1503 03.82 (P04C01)

## ANNEX TO THE EUROPEAN SEARCH REPORT
## ON EUROPEAN PATENT APPLICATION NO.

EP 21 18 3799

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2021

| Patent document cited in search report | | Publication date | Patent family member(s) | | Publication date |
|---|---|---|---|---|---|
| US 7554673 | B2 | 30-06-2009 | EP | 1953528 A1 | 06-08-2008 |
| | | | JP | 5242183 B2 | 24-07-2013 |
| | | | JP | 2008191152 A | 21-08-2008 |
| | | | US | 2008186504 A1 | 07-08-2008 |
| WO 9621140 | A1 | 11-07-1996 | CA | 2205875 A1 | 11-07-1996 |
| | | | DE | 69530225 T2 | 19-02-2004 |
| | | | EP | 0800643 A1 | 15-10-1997 |
| | | | JP | H10511772 A | 10-11-1998 |
| | | | US | 5550373 A | 27-08-1996 |
| | | | WO | 9621140 A1 | 11-07-1996 |
| US 2020378892 | A1 | 03-12-2020 | US | 2020378892 A1 | 03-12-2020 |
| | | | WO | 2020243341 A2 | 03-12-2020 |

EPO FORM P0459

For more details about this annex : see Official Journal of the European Patent Office, No. 12/82